# EUROPEAN PATENT APPLICATION

(11) **EP 3 888 611 A1**
(43) Date of publication of application: **06.10.2021**
(21) Application number: 21166340.6
(22) Date of filing: 31.03.2021
(51) Int. Cl.: A61G 10/00, F24F 3/163, F24F 8/00, F24F 8/108, F24F 8/167, F24F 8/22

(54) **AIR PURIFYING APPARATUS FOR PURIFYING AIR IN AN ISOLATION SPACE**

(30) Priority: 31.03.2020 US 202063002372 P; 09.02.2021 TW 110105049
(71) Applicant: Yao I Fabric Co., Ltd., Changhua County 508 (TW); Dong Guan Aconic Fabric Co., Ltd, Guangdong Province 523525 (CN)
(72) Inventor: Huang, Wei-Heng, 330 Taoyuan City (TW); Wang, Tsan-Ching, 508 Changhua County (TW)
(74) Representative: Straus, Alexander

(57) **Abstract**

An air purifying apparatus (2, 2', 2", 2"') for purifying air in an isolation space (IS) is disclosed. The isolation space (IS) is surrounded by a mask body (19) and bounded from an outer space (OS) by the mask body (19). The air purifying apparatus (2, 2', 2", 2"') includes a main casing (20, 20'), an intake device (21) and a decontamination device (22, 22'). The main casing (20, 20') is formed with an accommodating space (201), an inner exhausting runner (202) and an inner intake runner (203). The inner exhausting runner (202) is communicated with the isolation space (IS) and the accommodating space (201). The inner intake runner (203) is communicated with isolation space (IS) and the accommodating space (201). The intake device (21) and the decontamination device (22, 22') are disposed in the accommodating space (201).

## Description

### Field of the Invention

The present invention relates to an air purifying apparatus, and more particularly, to an air purifying device for purifying air in an isolation space.

### Background of the Invention

Generally speaking, therein no isolation facilities for hospital beds to form isolation space around the hospital beds. Therefore, the hospital beds cannot provide air disinfection and purification treatment to the space around the beds. Under the circumstance of limited medical resources in hospitals, multiple hospital beds are often set up in a ward to provide multiple patients for their hospitalization. In the absence of isolation space and the inability to provide air disinfection and purification treatment to the space around the bed, for multiple patients in the same ward, there is a risk of cross-infection during hospitalization.

### Summary of the Invention

This in mind, the present invention aims at providing an air purifying device for purifying air in an isolation space.

This is achieved by an air purifying device according to claims 1, 14 and 16. The dependent claims pertain to corresponding further developments and improvements.

As will be seen more clearly from the detailed description following below, the claimed air purifying apparatus for purifying air in an isolation space is disclosed. The isolation space is surrounded by a mask body. The isolation space and an outer space are bounded by the mask body. The air purifying apparatus includes a main casing, an intake device and a decontamination device. The main casing has an accommodating space, an inner exhausting runner and an inner intake runner formed therein. The inner exhausting runner is communicated with the isolation space and a side of the accommodating space. The inner intake runner is communicated with the isolation space and another side of the accommodating space. A cross-sectional area of the inner exhausting runner is different from a cross-sectional area of the inner intake runner. The intake device is disposed in the accommodating space. The decontamination device is disposed in the accommodating space. The decontamination device is disposed between the intake device and the inner exhausting runner and for purifying and disinfecting air in the accommodating space.

As will be seen more clearly from the detailed description following below, the claimed air purifying apparatus for purifying air in an isolation space is disclosed. The isolation space is surrounded by a mask body. The isolation space and an outer space are bounded by the mask body. The air purifying apparatus includes a main casing, an intake device and a decontamination device. The main casing has an accommodating space, an outer exhausting runner and an inner intake runner formed therein. The inner exhausting runner is communicated with the isolation space and a side of the accommodating space. The inner intake runner is communicated with the isolation space and another side of the accommodating space. A cross-sectional area of the inner exhausting runner is different from a cross-sectional area of the inner intake runner. The intake device is disposed in the accommodating space. The decontamination device is disposed in the accommodating space. The decontamination device is disposed between the intake device and the outer exhausting runner and for purifying and disinfecting air in the accommodating space.

As will be seen more clearly from the detailed description following below, the claimed air purifying apparatus for purifying air in an isolation space is disclosed. The isolation space is surrounded by a mask body. The isolation space and an outer space are bounded by the mask body. The air purifying apparatus includes a main casing, an intake device and a decontamination device. The main casing has an accommodating space, an inner exhausting runner and an outer intake runner formed therein. The inner exhausting runner is communicated with the isolation space and a side of the accommodating space. The inner intake runner is communicated with the isolation space and another side of the accommodating space. A cross-sectional area of the inner exhausting runner is different from a cross-sectional area of the inner intake runner. The intake device is disposed in the accommodating space. The decontamination device is disposed in the accommodating space. The decontamination device is disposed between the intake device and the outer exhausting runner and for purifying and disinfecting air in the accommodating space.

### Brief Description of the Drawings

In the following, the invention is further illustrated by way of example, taking reference to the accompanying drawings thereof:
FIG. 1 is an exploded diagram of a bed set according to a first embodiment of the present invention,
FIG. 2 is a diagram of an isolation mask according to the first embodiment of the present invention,
FIG. 3 is a diagram of the isolation mask in another view according to the first embodiment of the present invention,
FIG. 4A and FIG. 4B are diagrams of a supporting frame set respectively in two conditions according to the first embodiment of the present invention,
FIG. 5 is a diagram of the bed set in use according to the first embodiment of the present invention,
FIG. 6 is a diagram of the bed set in use in another view according to the first embodiment of the present invention,
FIG. 7 is a diagram of the bed set in another view according to the first embodiment of the present invention,
FIG. 8 is a diagram of a bed set according to a second embodiment of the present invention,
FIG. 9 is a diagram of the bed set in another view according to the second embodiment of the present invention,
FIG. 10 is a sectional diagram of a mattress pad according to another embodiment of the present invention,
FIG. 11 is a diagram of a bed set according to a third embodiment of the present invention,
FIG. 12 is a diagram of a ball valve device according to an embodiment of the present invention,
FIG. 13 is a diagram of a pin valve device according to an embodiment of the present invention,
FIG. 14 is a diagram of a bed with a decontamination device according to a yet embodiment of the present invention,
FIG. 15 is a top view of a bed set according to a fourth embodiment of the present invention,
FIG. 16 is a diagram of a bed set according to a fifth embodiment of the present invention,
FIG. 17 is a front view of a bed set according to a sixth embodiment of the present invention, and
FIG. 18 is a front view of a bed set according to a seventh embodiment of the present invention.

### Detailed Description

In order to enable the skilled persons in the art to better understand the present disclosure, hereinafter preferred embodiments with drawings are provided for illustrating the present disclosure and the effect to be achieved. It should be noted that the drawings are simplified schematic diagrams. Therefore, only elements related to the present disclosure and combination relationship thereof are shown to provide a clearer description of the basic framework or implementation methods of the present disclosure. The actual elements and configuration may be more complicated. In addition, for the sake of convenience, the number of the components in the drawings could be unequal the actual number thereof, the shape and size of the components may not draw in proportion to the actual shape and size, and the proportion thereof can be adjusted according to design requirements.

The directional terminology in the following embodiments, such as top, bottom, left, right, front or back, is used with reference to the orientation of the Figure(s) being described. As such, the directional terminology is used for purposes of illustration and is in no way limiting.

The ordinal number terminology, such as first, second and third, can be used to describe various elements, and the elements are not limited by definition of the ordinal number terminology. The ordinal number terminology is used to distinguish one element from other elements in the specification, and the ordinal number terminology of the element in the claims is arranged according to the claimed order and could be different from that in the specification. As such, a first element recited in the following description could be a second element in the claims.

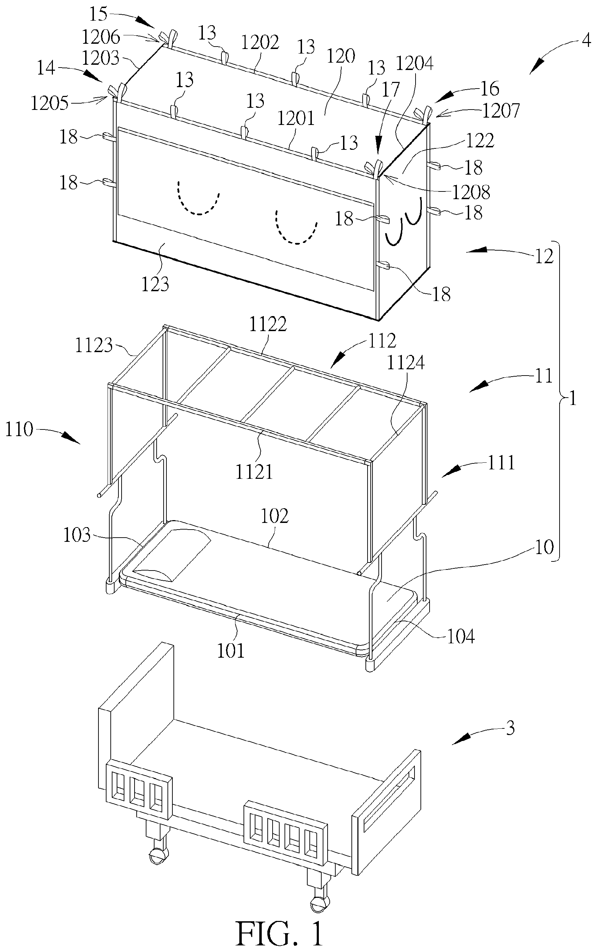

Please refer to FIG. 1. FIG. 1 is an exploded diagram of a bed set 4 according to a first embodiment of the present invention. The bed set 4 includes a bed 3 and an isolation assembly 1. The isolation assembly 1 is detachably installed on the bed 3. The isolation assembly 1 includes a mattress pad 10, a supporting frame set 11 and an isolation mask 12 (or mask body). The mattress pad 10 is installed on the bed 3. The supporting frame set 11 is installed on the bed 3. The isolation mask 12 is installed on the supporting frame set 11.

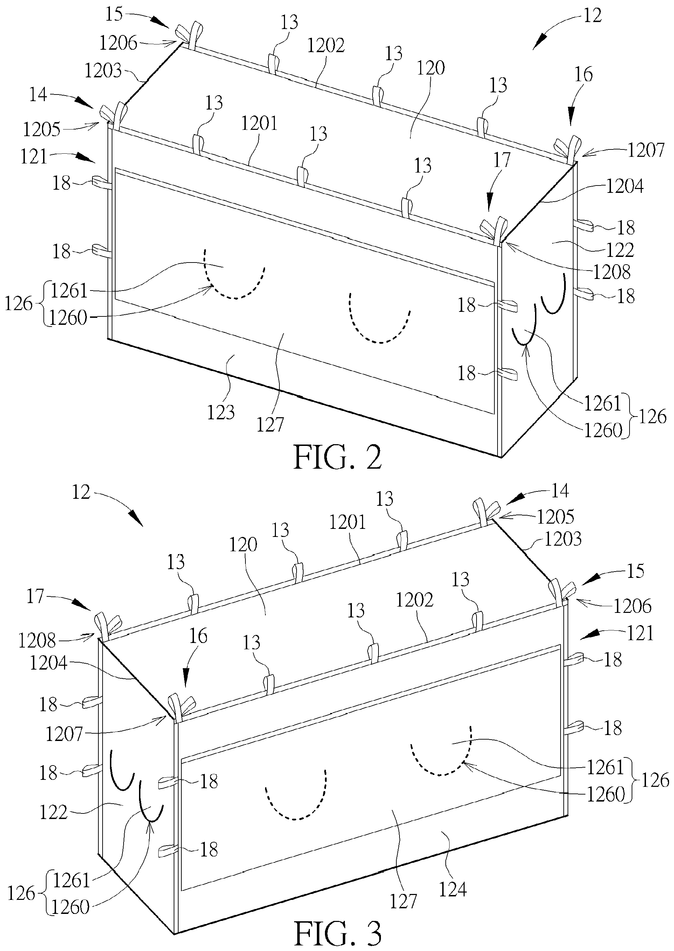

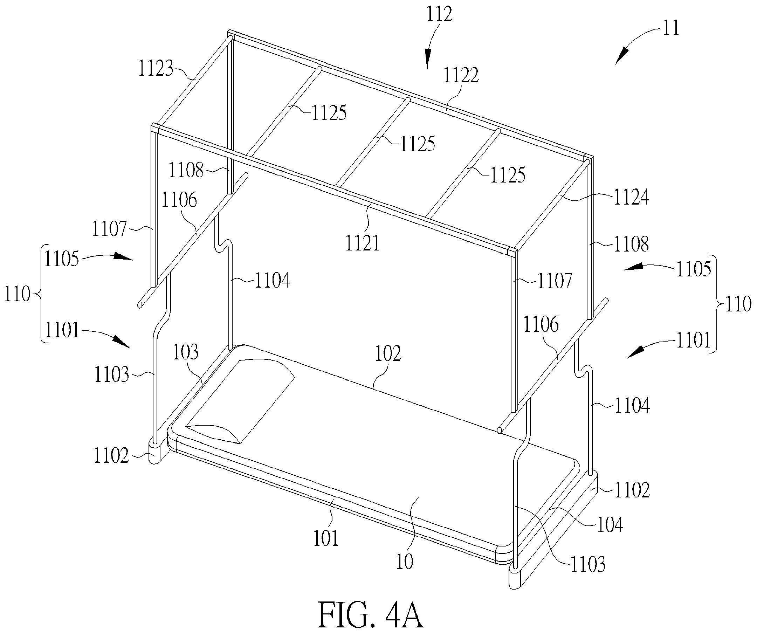

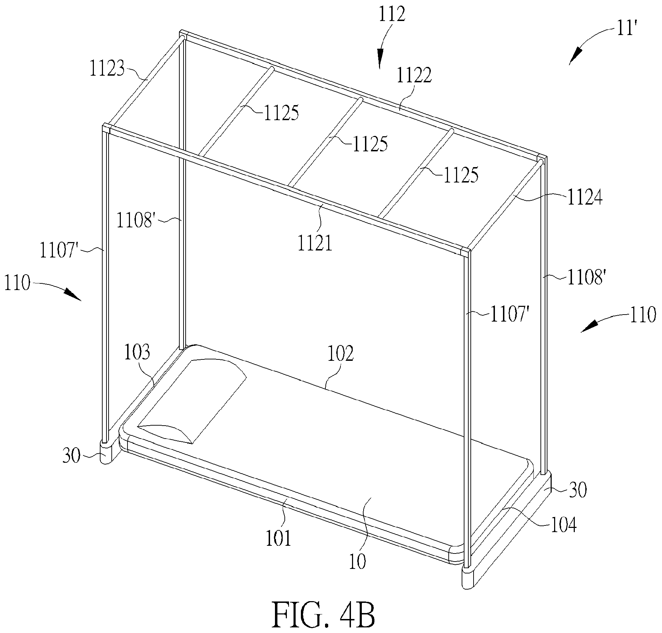

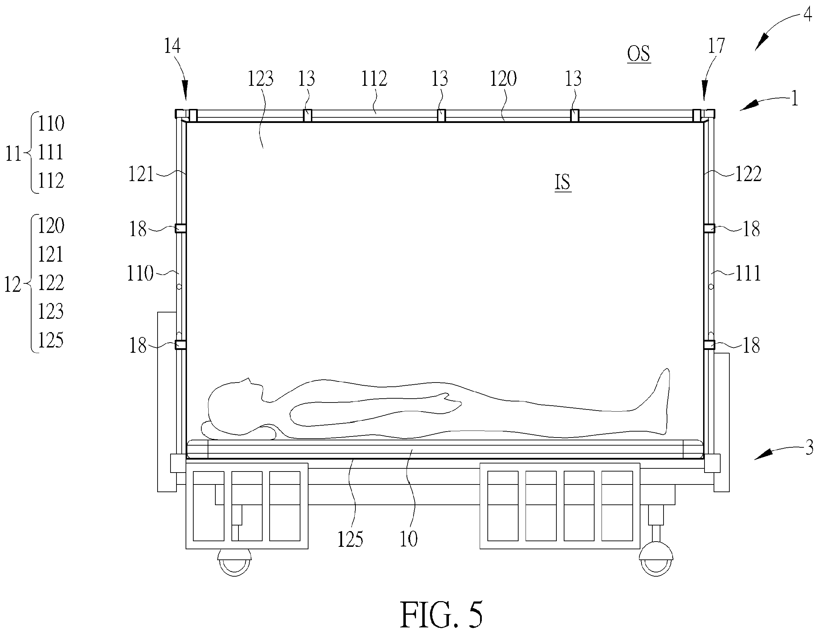

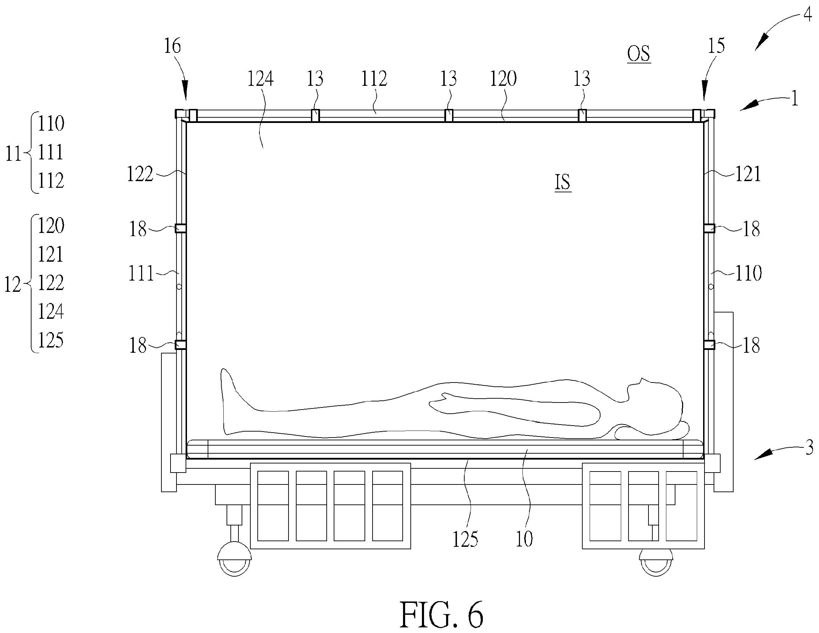

Please refer FIG. 1 to FIG. 6. FIG. 2 is a diagram of the isolation mask 12 according to the first embodiment of the present invention. FIG. 3 is a diagram of the isolation mask 12 in another view according to the first embodiment of the present invention. FIG. 4A and FIG. 4B are diagrams of the supporting frame set 11 respectively in two conditions according to the first embodiment of the present invention. FIG. 5 is a diagram of the bed set 4 in use according to the first embodiment of the present invention. FIG. 6 is a diagram of the bed set 4 in use in another view according to the first embodiment of the present invention. The supporting frame set 11 includes a first lateral frame set 110, a second lateral frame set 111 and a top frame set 112. The first lateral frame set 110 is disposed on the bed 3 and adjacent to a side of the mattress pad 10. The second lateral frame set 111 is disposed on the bed 3 and adjacent to another side of the mattress pad 10. The top frame set 112 is disposed on the first lateral frame set 110 and the second lateral frame set 111. The isolation mask 12 includes a top portion 120, a head portion 121, a foot portion 122, a first lateral door portion 123 and a second lateral door portion 124. The head portion 121 and the foot portion 122 are respectively connected to opposite sides of the top portion 120 and face each other. The first lateral door portion 123 and the second lateral door portion 124 are respectively connected to opposite sides of the top portion 120 and face each other.

Furthermore, the top frame set 112 holds the top portion 120 of the isolation mask 12, such that the head portion 121, the foot portion 122, the first lateral door portion 123 and the second lateral door portion 124 of the isolation mask 12 are located around a periphery of the mattress pad 10, so as to form an isolation space IS for the mattress pad 10 to be accommodated. In practical application, the bed 3 can be a hospital bed, and the bed 3 may contain the mattress originally set on the hospital bed (not shown in figures), and the supporting frame set 11 of the isolation assembly 1 is installed on the hospital bed (i.e., the bed 3) for holding or supporting the isolation mask 12, so that the isolation mask 12 forms the isolation space IS above and around the hospital bed (i.e., the bed 3). In other words, the isolation space IS is surrounded by at least the mask body (i.e., the isolation mask 12), and the isolation space IS and an outer space OS are bounded by the mask body (i.e., the isolation mask 12).

In such a manner, the isolation assembly 1 of the present invention is able to be adapted to the hospital bed (i.e., the bed 3) and provides a patient with the isolation space IS for preventing the patient from bothering or allowing the patient to be treated in the isolation space IS constructed by the isolation assembly 1. The application of the isolation assembly 1 is not limited to the hospital bed, e.g., the isolation assembly 1 can be adapted to a home bed for providing an isolation space at home, or alternatively, the isolation assembly 1 can be adapted to an outdoor bed for providing an isolation space in the outdoor field.

Furthermore, the isolation mask 12 includes a first borderline 1201, a second borderline 1202, a third borderline 1203 and a fourth borderline 1204. The first lateral door portion 123 is extended from the first borderline 1201. The second lateral door portion 124 is extended from the second borderline 1202. The head portion 121 is extended from the third borderline 1203. The foot portion 122 is extended from the fourth borderline 1204. The top frame set 112 of the supporting frame set 11 includes a first beam 1121, a second beam 1122, a third beam 1123 and a fourth beam 1124. The first beam 1121 is connected to a side of the first lateral frame set 110 and a side of the second lateral frame set 111. The first beam 1121 holds the first borderline 1201 of the top portion 120, such that the first lateral door portion 123 is located on a first side 101 of the mattress pad 10. The second beam 1122 is connected to another side of the first lateral frame set 110 and another side of the second lateral frame set 111. The second beam 1122 holds the second borderline 1202 of the top portion 120, such that the second lateral door portion 124 is located on a second side 102 of the mattress pad 10. The third beam 1123 is connected to an end of the first beam 1121 and an end of the second beam 1122. The third beam 1123 holds the third borderline 1203 of the top portion 120, such that the head portion 121 is located on a third side 103 of the mattress pad 10. The fourth beam 1124 is connected to another end of the first beam 1121 and another end of the second beam 1122. The fourth beam 1124 holds the fourth borderline 1204 of the top portion 120, such that the foot portion 122 is located on a fourth side 104 of the mattress pad 10.

In the embodiment, the top frame set 112 of the supporting frame set 11 further includes a plurality of a plurality of crossbeams 1125. The plurality of crossbeams 1125 are respectively connected to the first beam 1121 and the second beam 1122 and for enhancing overall structural strength of the top frame set 112 of the supporting frame set 11, such that the top frame set 112 is able to support the top portion 120 of the isolation mask 12 more stably. An amount of the crossbeam 1125 is not limited to those illustrated in figures in the embodiment. The top frame set 112 of the supporting frame set 11 including more than one crossbeam 1125 is within the scope of the present invention. In addition, the crossbeam(s) 1125 of the present invention can be omitted, i.e., in the embodiment, the top frame set 112 of the supporting frame set 11 can include crossbeam(s) 1125. In another embodiment, the top frame set 112 of the supporting frame set 11 can omit the crossbeam(s) 1125. In other words, disposal of the crossbeam(s) 1125 depends on requirement of the structural strength.

In the embodiment, the first lateral frame set 110 and the second lateral frame set 111 of the supporting frame set 11 have identical structures. Hereinafter, the first lateral frame set 110 is illustrated as an example. The second lateral frame set 111 can be deducted, and related descriptions of the second lateral frame set 111 are omitted herein for simplicity. As shown in FIG. 1 to FIG. 6, the first lateral frame set 110 of the supporting frame set 11 includes a connecting frame 1101 and a supporting frame 1105. The connecting frame 1101 is connected to the bed 3, and the supporting frame 1105 is connected to the connecting frame 1101 and the top frame set 112.

Furthermore, the supporting frame 1105 includes a cross bar 1106, a first straight bar 1107 and a second straight bar 1108. The first straight bar 1107 is connected to an end of the cross bar 1106 and a side of the top frame set 112. The second straight bar 1108 is connected to another end of the cross bar 1106 of another side of the top frame set 112. The connecting frame 1101 includes a first connecting bar 1103 and a second connecting bar 1104. Two ends of the first connecting bar 1103 are respectively connected to an end of the bed 3 and an end portion of the cross bar 1106. Two ends of the second connecting bar 1104 are respectively connected to another end of the bed 3 and another end of the cross bar 1106. Furthermore, the connecting frame 1101 further includes a pivot bar 1102 pivoted to the bed 3. Two ends of the pivot bar 1102 are respectively connected to an end of the first connecting bar 1103 and an end of the second connecting bar 1104.

In practical application, at least one of a combination of the first straight bar 1107 and the second straight bar 1108 and a combination of the first connecting bar 1103 and the second connecting bar 1104 can be a stretchable linkage assembly. The stretchable linkage assembly (i.e., the combination of the first straight bar 1107 and the second straight bar 1108 and/or the combination of the first connecting bar 1103 and the second connecting bar 1104) is able to adjust a distance between the top frame set 112 and the bed 3 by stretchable design thereof, so as to satisfy requirements of space for various patients.

It is noticed that the first lateral frame set 110 of the present invention includes two sets of frame, i.e., the connecting frame 1101 and the supporting frame 1105. The connecting frames 1101 with same size and the supporting frame 1105 with alternative size are utilized for the bed 3 with different sizes. Furthermore, the first lateral frame set 110 of the present invention is detachably installed on the bed 3 by the pivot bar 1102 of the connecting frame 1101. Structures of the first lateral frame set 110 of the present invention are not limited to those mentioned above. For example, the first lateral frame set 110 of the present invention can include only one of the connecting frame 1101 and the supporting frame 1105, and is detachably installed on the bed 3 by the one of the connecting frame 1101 and the supporting frame 1105. As for which design is adopted, it depends on practical demands.

For example, please refer to FIG. 4B. FIG. 4B is a diagram of a supporting frame set 11' according to another embodiment of the present invention. A major difference between the supporting frame set 11' and the above-mentioned supporting frame set 11 is that the supporting frame set 11' includes a first straight bar 1107' and a second straight bar 1108' only, wherein the first straight bar 1107' is connected to a holding base 30 of the bed 3 and a side of the top frame set 112, and the second straight bar 1108' is connected to the holding base 30 of the bed 3 and another side of the top frame set 112. In the embodiment, the holding base 30 of the bed 3 can be a drip stand holder, i.e., an end of the first straight bar 1107' and an end of the second straight bar 1108' are respectively inserted into two installing holes of the drip stand holder (i.e., the holding base 30). In order for better support, diameters of the first straight bar 1107' and the second straight bar 1108' can be slightly larger or smaller than bore sizes of the installing holes. When the bore size of one of the two installing holes of the drip stand holder does not match the diameter of the first straight bar 1107' or the second straight bar 1108', an adapter (not shown in figures) can be utilized with one end being inserted into the installing hole of the drip stand holder and one end being connected to the straight bar. In another embodiment, the first connecting bar 1103 and the second connecting bar 1104 of the connecting frame 1101 can also be respectively inserted into the two installing holes of the drip stand holder (i.e., the holder 30). When the bore size of one of the two installing holes does not match the diameter of the first connecting bar 1103 or the second connecting bar 1104, a tight connection can be provided through the adapter.

Namely, the first straight bar 1107' and the second straight bar 1108' can be respectively a stretchable linkage assembly. The stretchable linkage assembly (i.e., the first straight bar 1107' and the second straight bar 1108') is able to adjust a distance between the top frame set 112 and the bed 3 by stretchable design thereof, so as to satisfy requirements of space for various patients.

As shown in FIG. 1 to FIG. 6, the application field of the present invention may be a medical institution or a nursing center. For the sake of easy assembly, the isolation mask 12 is installed on an interior of the supporting frame set 11, such that the isolation mask 12 is located between the mattress pad 10 and the supporting frame set 11. In addition, for enhancing isolation effect, the isolation mask 12 further includes a bottom portion 125 located between the mattress pad 10 and the bed 3. Four sides of the bottom portion 125 are respectively connected to the first lateral door portion 123, the second lateral door portion 124, a head portion 121 and the foot portion 122. The isolation space IS is further defined by the top portion 120, the head portion 121, the foot portion 122, the first lateral door portion 123, the second lateral door portion 124 and the bottom portion 125. In other words, in the embodiment, the isolation mask 12 is an enclosed box formed by the top portion 120, the head portion 121, the foot portion 122, the first lateral door portion 123, the second lateral door portion 124 and the bottom portion 125, and the isolation space IS is an internal space of the enclosed box.

As mentioned above, since the isolation mask 12 is installed on the interior of the supporting frame set 11 and located between the mattress pad 10 and the supporting frame set 11, the isolation assembly 1 of the embodiment can further includes a plurality of top frame fixing members 13. The plurality of top frame fixing members 13 is for connecting the first borderline 1201 of the top portion 120 and the first beam 1121 of the top frame set 112, the second borderline 1202 of the top portion 120 and the second beam 1122 of the top frame set 112, the third borderline 1203 of the top portion 120 and the third beam 1123 of the top frame set 112 and the fourth borderline 1204 of the top portion 120 and the fourth beam 1124 of the top frame set 112.

In such a manner, the first borderline 1201, the second borderline 1202, the third borderline 1203 and the fourth borderline 1204 of the top portion 120 of the isolation mask 12 are respectively connected to the first beam 1121, the second beam 1122, the third beam 1123 and the fourth beam 1124 of the top frame set 112 through the plurality of top frame fixing members 13, such that the first beam 1121, the second beam 1122, the third beam 1123 and the fourth beam 1124 of the top frame set 112 are able to support the top portion 120 of the isolation mask 12, resulting in that the top portion 120 of the isolation mask 12 is hung above the mattress pad 10.

Furthermore, the isolation assembly 1 can further include a plurality of the lateral frame fixing members 18. The plurality of the lateral frame fixing members 18 are configured to connect the head portion 121 and the first lateral door portion 123 of the isolation mask 12 to a side of the first lateral frame set 110, such that the head portion 121 and the first lateral door portion 123 of the isolation mask 12 are fixed on the side of the first lateral frame set 110; connect the head portion 121 and the second lateral door portion 124 of the isolation mask 12 to another side of the first lateral frame set 110, such that the head portion 121 and the second lateral door portion 124 of the isolation mask 12 are fixed on the another side of the first lateral frame set 110; connect the foot portion 122 and the first lateral door portion 123 of the isolation mask 12 to a side of the second lateral frame set 111, such that the foot portion 122 and the first lateral door portion 123 of the isolation mask 12 are fixed on the side of the second lateral frame set 111; and connect the foot portion 122 and the second lateral door portion 124 of the isolation mask 12 to another side of the second lateral frame set 111, such that the foot portion 122 and the second lateral door portion 124 of the isolation mask 12 are fixed on the another side of the second lateral frame set 111.

In such a manner, the head portion 121, the foot portion 122, the first lateral door portion 123 and the second lateral door portion 124 of the isolation mask 12 are respectively connected to the first lateral frame set 110 and the second lateral frame set 111 through the plurality of lateral frame fixing members 18, such that the first lateral frame set 110 and the second lateral frame set 111 are able to support the head portion 121, the foot portion 122, the first lateral door portion 123 and the second lateral door portion 124 of the isolation mask 12 are fixedly located around the mattress pad 10.

In addition, at least one of the top portion 120, the head portion 121, the foot portion 122, the first lateral door portion 123 and the second lateral door portion 124 of the isolation mask 12 has a cross portion 126. In the embodiment, all the top portion 120, the head portion 121, the foot portion 122, the first lateral door portion 123 and the second lateral door portion 124 of the isolation mask 12 have the cross portion 126. An amount and position(s) of the cross portion 126 are not limited to those illustrated in figures in the embodiment. The cross portion 126 is provided to allow a limb to enter the isolation space IS. For example, the cross portion 126 allows the hands of medical staff or physicians to enter the isolation space IS for medical treatment or diagnosis of patients in the isolation space IS.

In the embodiment, the cross portion 126 comprises an opening 1260 and a covering body 1261. The opening 1260 is formed on at least one of the top portion 120, the head portion 121, the foot portion 122, the first lateral door portion 123 and the second lateral door portion 124 of the isolation mask 12. In the embodiment, all the top portion 120, the head portion 121, the foot portion 122, the first lateral door portion 123 and the second lateral door portion 124 of the isolation mask 12 have the opening 1260 for allowing the limb to enter the isolation space IS. An amount and disposal positions of the opening 1260 of the present invention is not limited to those mentioned in figures in the embodiment. The covering body 1261 is extended from an edge of the opening 1260 and for covering the opening 1260, so as to reduce insufficient sealing of the isolation space IS due to the design of the opening 1260.

In addition, the isolation mask 12 further has at least one curtain portion 127. The at least one curtain portion 127 is for covering the opening 1260 of the cross portion 126, so as to further reduce the insufficient sealing of the isolation space IS due to the design of the opening 1260. In the embodiment, the isolation mask 12 has two curtain portions 127, which are respectively disposed on the first lateral door portion 123 and the second lateral door portion 124. An amount and disposal positions of the curtain portion 127 are not limited to those illustrated in figures in the embodiment, e.g., the isolation mask 12 may has four curtain portions 127, and the four curtain portions 127 may be disposed on the head portion 121, the foot portion 122, the first lateral door portion 123 and the second lateral door portion 124 based on practical demands.

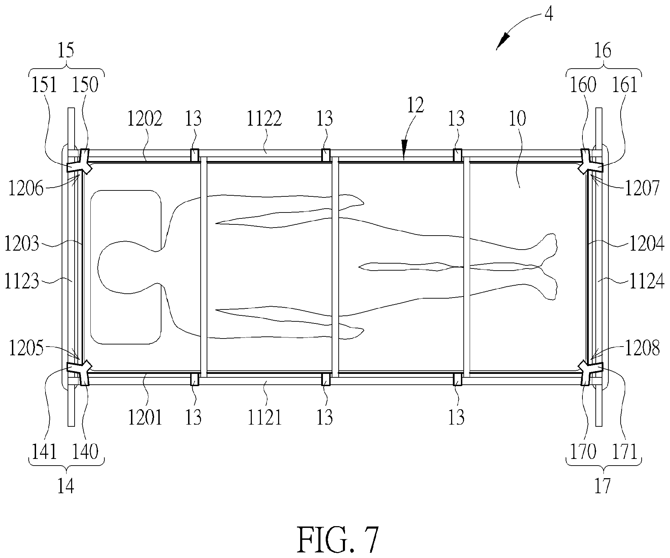

Please refer to FIG. 7. FIG. 7 is a diagram of the bed set 4 in another view according to the first embodiment of the present invention. A joint of the first borderline 1201 and the third borderline 1203 of the top portion 120 defines a first corner 1205. The isolation assembly 1 can further include a first corner fixing member 14 disposed on the first corner 1205. The first corner fixing member 14 has a first fixing portion 140 and a second fixing portion 141. The first fixing portion 140 of the first corner fixing member 14 is for being connected to the first beam 1121, and the second fixing portion 141 of the first corner fixing member 14 is for being connected to the third beam 1123. As a result, the first corner fixing member 14 is able to constrain displacement of the first corner 1205 of the top portion 120 of the isolation mask 12 with respect to the first beam 1121 and the third beam 1123, so as to prevent the first corner 1205 of the top portion 120 of the isolation mask 12 from over-displacement with respect to the first beam 1121 and the third beam 1123.

A joint of the third borderline 1203 and the second borderline 1202 of the top portion 120 of the isolation mask 12 defines a second corner 1206. The isolation assembly 1 can further include a second corner fixing member 15 disposed on the second corner 1206. The second corner fixing member 15 has a first fixing portion 150 and a second fixing portion 151. The first fixing portion 150 of the second corner fixing member 15 is for being connected to the second beam 1122, and the second fixing portion 151 of the second corner fixing member 15 is for being connected to the third beam 1123. As a result, the second corner fixing member 15 is able to constrain displacement of the second corner 1206 of the top portion 120 of the isolation mask 12 with respect to the second beam 1122 and the third beam 1123, so as to prevent the second corner 1206 of the top portion 120 of the isolation mask 12 from over-displacement with respect to the second beam 1122 and the third beam 1123.

A joint of the second borderline 1202 and the fourth borderline 1204 of the top portion 120 of the isolation mask 12 defines a third corner 1207. The isolation assembly 1 can further include a third corner fixing member 16 disposed on the third corner 1207. The third corner fixing member 16 has a first fixing portion 160 and a second fixing portion 161. The first fixing portion 160 of the third corner fixing member 16 is for being connected to the second beam 1122, and the second fixing portion 161 of the third corner fixing member 16 is for being connected to the fourth beam 1124. As a result, the third corner fixing member 16 is able to constrain displacement of the third corner 1207 of the top portion 120 of the isolation mask 12 with respect to the second beam 1122 and the fourth beam 1124, so as to prevent the third corner 1207 of the top portion 120 of the isolation mask 12 from over-displacement with respect to the second beam 1122 and the fourth beam 1124.

A joint of the fourth borderline 1204 and the first borderline 1201 of the top portion 120 of the isolation mask 12 defines a fourth corner 1208. The isolation assembly 1 can further include a fourth corner fixing member 17 disposed on the fourth corner 1208. The fourth corner fixing member 17 has a first fixing portion 170 and a second fixing portion 171. The first fixing portion 170 of the fourth corner fixing member 17 is for being connected to the first beam 1121, and the second fixing portion 171 of the fourth corner fixing member 17 is for being connected to the fourth beam 1124. As a result, the fourth corner fixing member 17 is able to constrain displacement of the fourth corner 1208 of the top portion 120 of the isolation mask 12 with respect to the first beam 1121 and the fourth beam 1124, so as to prevent the fourth corner 1208 of the top portion 120 of the isolation mask 12 from over-displacement with respect to the first beam 1121 and the fourth beam 1124.

In the embodiment, the isolation assembly 1 includes the first corner fixing member 14, the second corner fixing member 15, the third corner fixing member 16 and the fourth corner fixing member 17. An amount and disposal positions of the corner fixing member are not limited to those illustrated in figures in the embodiment. For example, the isolation assembly 1 may include at least two aforesaid corner fixing members, which are disposed on two diagonal corners defined by the first borderline 1201, the second borderline 1202, the third borderline 1203 and the fourth borderline 1204. In one embodiment, the structure that the isolation assembly 1 includes at least two corner fixing members, e.g., the first corner fixing member 14 and the third corner fixing member 16, may constrain the displacement of the four corners of the top portion 120 of the isolation mask 12 with respect to the four beams for preventing the over-displacement of the isolation mask 12. In another embodiment, the structure that the isolation assembly 1 includes at least two corner fixing members, e.g., the second corner fixing member 15 and the fourth corner fixing member 17, may constrain the displacement of the four corners of the top portion 120 of the isolation mask 12 with respect to the four beams for preventing the over-displacement of the isolation mask 12.

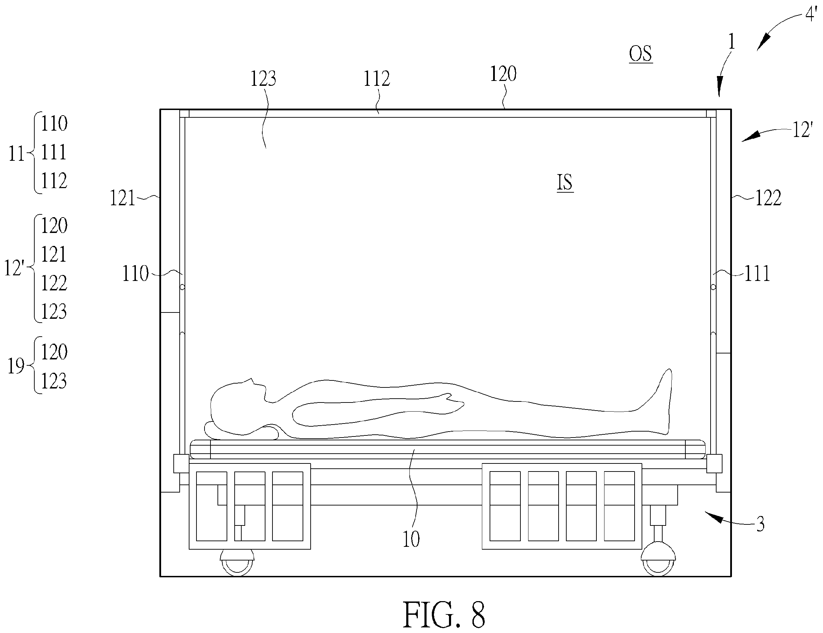

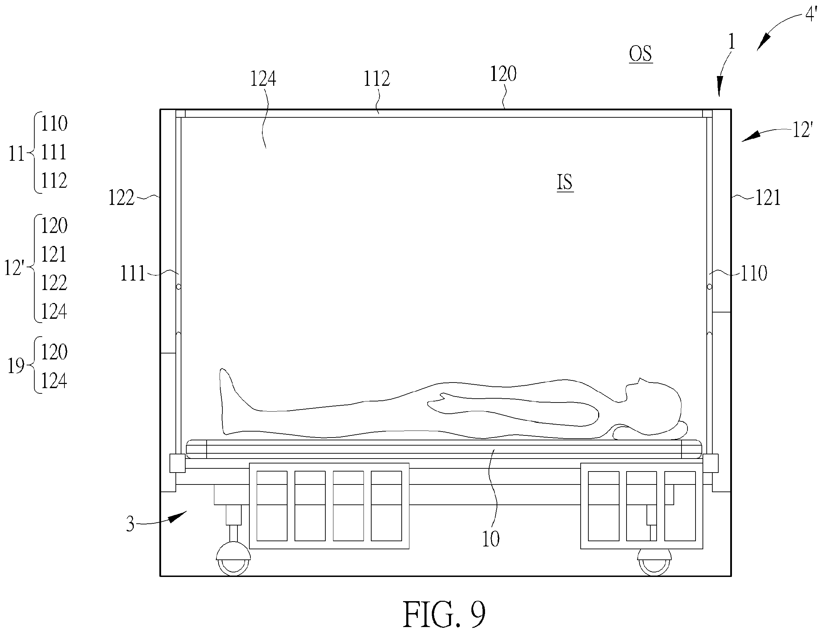

Please refer to FIG. 8 and FIG. 9. FIG. 8 is a diagram of a bed set 4' according to a second embodiment of the present invention. FIG. 9 is a diagram of the bed set 4' in another view according to the second embodiment of the present invention. A major difference between the bed set 4' and the aforesaid bed set 4 is that an isolation mask 12' of the bed set 4' is installed on an external side of the supporting frame set 11, such that the supporting frame set 11 is located between the isolation mask 12' and the mattress pad 10. In other words, since the isolation mask 12' of the bed set 4' is installed on the external side of the supporting frame set 11, relative position between the isolation mask 12' and the supporting frame set 11 is confirmed by the isolation mask 12' sheathing the supporting frame set 11. As a result, fixing members can be omitted for the isolation mask 12' for simplifying manufacture process and providing a wider space both for vision and accommodation for the isolation mask 12'.

Furthermore, the isolation mask 12' does not have a bottom portion, and a top portion 120, a first lateral door portion 123 and a second lateral door portion 124 of the isolation mask 12' forms a mask body 19. The head portion 121 of the isolation mask 12' is detachably installed on the mask body 19, and the foot portion 122 of the isolation mask 12' is detachably installed on the mask body 19. In practical application, the head portion 121 and the foot portion 122 of the isolation mask 12' may be detachably installed on the mask body 19 in a zippered manner, but the present invention is not limited thereto.

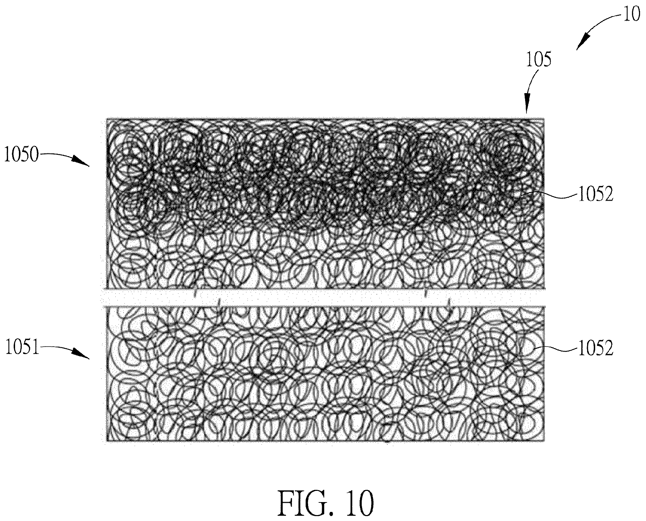

Please refer to FIG. 10. FIG. 10 is a sectional diagram of the mattress pad 10 according to another embodiment of the present invention. The mattress pad 10 has a mattress body 105, the mattress body 105 is an elastic three-dimensional structure made of a plurality of thermoplastic filaments and the plurality of thermoplastic filaments are entangled and contacted with each other. Overall appearance of the elastic three-dimensional structure is roughly rectangular and its interior is roughly irregular network. The above-mentioned thermoplastic filament 1052 has the characteristic that the expansion ratio increases with the shear rate. In practical application, molten plastic is put into an extruder (not shown in the figure) and then extrude into several thermoplastic filaments 1052, and the thermoplastic filaments 1052 are entangled and contact each other in an irregular manner, and then the molten thermoplastic filaments 1052 are connected and shaped into a staggered three-dimensional structure with considerable elasticity. The above-mentioned thermoplastic filament 1052 can be a solid or hollow wire body, the plurality of thermoplastic filaments 1052 are joined and fixed at a contact position and form pores at a non-contact position. Since the three-dimensional structure made of the thermoplastic filament 1052 is provided with high supportability, and the entire three-dimensional structure has a large number of penetrating pores therein, the three-dimensional structure has a relatively high porosity, so that air can be effectively contained in the three-dimensional structure. As a result, air flow and circulation is effectively generated in the three-dimensional structure, which allows the three-dimensional structure to have good air permeability.

It should be noted that, in order to allow the mattress pad 10 to be easily rolled up, entanglement of the thermoplastic filament 1052 inside the mattress pad 10 is designed to be dense and sparse. Specifically, the thermoplastic filaments 1052 within an upper portion 1050 of the mattress body 105 are irregularly entangled and contacted with each other in a dense manner, and the thermoplastic filaments 1052 within a lower portion 1051 are irregularly entangled and contacted with each other in a sparse manner. As a result, the porosity of the top portion 1050 of the mattress body 105 will be lower than the porosity of the lower portion 1051 of the mattress body 105, and the elasticity of the top portion 1050 will be lower than that of the lower portion 1051. In such a manner, the lower portion 1051 will be more elastic and easy to be bent, which is convenient for the user to easily roll up the mattress body 105 to reduce the volume when the mattress pad 10 needs to be transported.

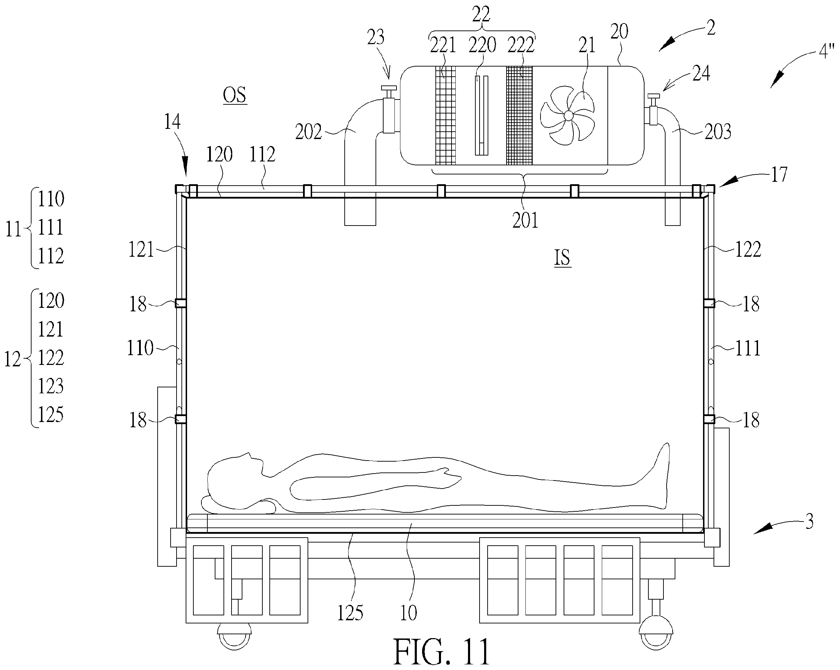

Please refer to FIG. 11. FIG. 11 is a diagram of a bed set 4" according to a third embodiment of the present invention. A major difference between the bed set 4" and the aforesaid bed set 4 is that the bed set 4" further includes an air purifying apparatus 2 for purifying air inside the isolation space IS, wherein the isolation space IS is surrounded by the mask body (i.e., the isolation mask 12), and the isolation space IS and an outer space OS is bounded by the mask body (i.e., the isolation mask 12).

Furthermore, the air purifying apparatus 2 includes a main casing 20, an intake device 21 and a decontamination device 22. The main casing 20 is formed with an accommodating space 201, an inner exhausting runner 202 and an inner intake runner 203. The inner exhausting runner 202 is communicated with the isolation space IS and a side of the accommodating space 201. The inner intake runner 203 is communicated with isolation space IS and another side of the accommodating space 201. The intake device 21 and the decontamination device 22 are disposed in the accommodating space 201. The decontamination device 22 is disposed between the intake device 21 and the inner exhausting runner 202 and for purifying and decontaminating air in the accommodating space 201.

In such a manner, when the air inside the isolation space IS flows into the accommodating space 201 via the inner exhausting runner 202, the air is purified and disinfected by the decontamination device 22 and then flew into the isolation space IS via the inner intake runner 203, so as to purify and disinfect the air in the isolation space IS. In other words, the air purifying apparatus 2 of the bed set 4" has a circulation mode of "inner-exhausting-inner-intake".

It is noticed that, in the embodiment, a cross-sectional area of the inner exhausting runner 202 is larger than a cross-sectional area of the inner intake runner 203. As a result, under a condition of the same intake device 21, an inner exhausting flux flew out of the isolation space IS via the inner exhausting runner 202 is larger than an inner intake flux flew into the isolation space IS via the inner intake runner 203. In another embodiment, the cross-sectional area of the inner exhausting runner 202 is smaller than the cross-sectional area of the inner intake runner 203. As a result, under the condition of the same intake device 21, the inner exhausting flux flew out of the isolation space IS via the inner exhausting runner 202 is smaller than the inner intake flux flew into the isolation space IS via the inner intake runner 203. In other words, the present invention is able to adjust the inner exhausting flux and the inner intake flux through a design that the cross-section of the inner exhausting runner 202 is different from the cross-section of the inner intake runner 203.

It is noticed that the decontamination device 22 is disposed between the intake device 21 and the inner exhausting runner 202 in the embodiment, which allows the air flew through the intake device 21 is purified and disinfected by the decontamination device 22 in advance, so as to reduce a degree of pollution to the air intake device 21 caused by the air flew through the air intake device 21.

Furthermore, the air purifying apparatus 2 further includes an inner intake flow adjustment device 23. The inner intake flow adjustment device 23 is coupled to the inner intake runner 203 and for adjusting the inner intake flux flew into the isolation space IS via the inner intake runner 203. In addition, the air purifying apparatus 2 can further include an inner exhausting flow adjustment device 24. The inner exhausting flow adjustment device 24 is coupled to the inner exhausting runner 202 and for adjusting the inner exhausting flux flew out from the isolation space IS via the inner exhausting runner 202.

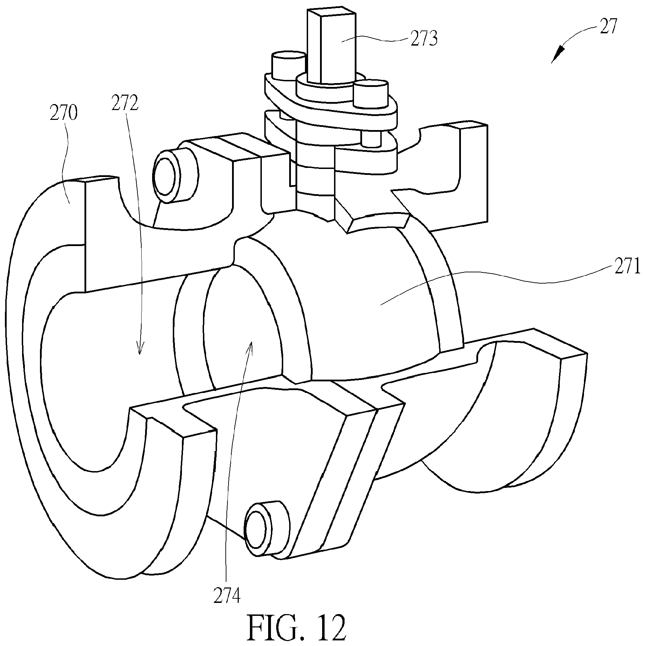

In the embodiment, at least one of the inner intake flow adjustment device 23 and the inner exhausting flow adjustment device 24 is a ball valve device or a butterfly valve device. Hereinafter, it is illustrated as an example that the at least one of the inner intake flow adjustment device 23 and the inner exhausting flow adjustment device 24 is the ball valve device. Please refer to FIG. 12. FIG. 12 is a diagram of a ball valve device 27 according to an embodiment of the present invention. The ball valve device 27 is disposed in at least one of the inner intake runner 203 and the inner exhausting runner 202 and includes a base 270 and a rotating valve member 271. The base 270 has a base flow runner 272 therein, and the rotating valve member 271 is disposed in the base flow runner 272. The rotating valve member 271 varies a cross-sectional area of the base flow runner 272 in a rotating manner, so as to further vary the cross-sectional area of the inner exhausting runner 202 and/or the cross-sectional area of the inner intake runner 203.

In the embodiment, the rotating valve member 271 is a ball member. The ball member is rotatably disposed in the base flow runner 272 and has a channel 274. The ball valve device 27 further includes a shaft member 273. The shaft member 273 is connected to the ball member and drives the ball member to rotate about an axis of the shaft member 273. In such a manner, when the shaft member 273 drives the ball member to rotate to a position shown in FIG. 12, the channel 274 is completely exposed to the base flow runner 272. When the shaft member 273 drives the ball member to rotate away from the position shown in FIG. 12, a part of the channel 274 is blocked by a wall of the base 270. As a result, the cross-sectional area of the base flow runner 272 is varied, so as to vary the cross-sectional area of the inner exhausting runner 202 and/or the cross-sectional area of the inner intake runner 203.

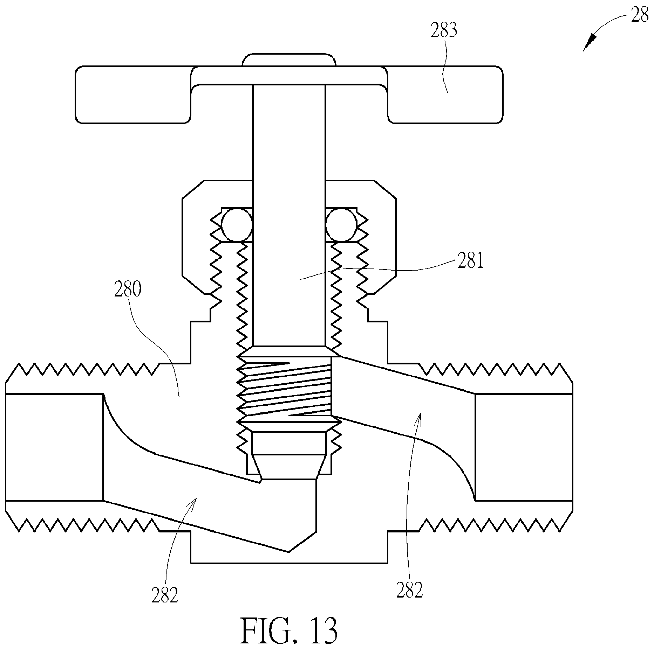

In another embodiment, at least one of the inner intake flow adjustment device 23 and the inner exhausting flow adjustment device 24 is a blade valve device or a pin valve device. Hereinafter, it is illustrated as an example that the at least one of the inner intake flow adjustment device 23 and the inner exhausting flow adjustment device 24 is the pin valve device. Please refer to FIG. 13. FIG. 13 is a diagram of a pin valve device 28 according to an embodiment of the present invention. The pin valve device 28 is disposed in at least one of the inner intake runner 203 and the inner exhausting runner 202 and includes a base 280 and a linear valve member 281. The base 280 has a base flow runner 282 therein, and the linear valve member 281 is disposed in the base flow runner 282. The linear valve member 281 varies a cross-sectional area of the base flow runner 282 in a linearly moving manner, so as to further vary the cross-sectional area of the inner exhausting runner 202 and/or the cross-sectional area of the inner intake runner 203.

In the embodiment, the linear valve member 281 is a screw. The screw is screwedly connected to the base 280, and the pin valve device 28 further has a handle 283. The handle 283 is connected to the screw and drives the screw to rotate about an axis thereof. In such a manner, when the handle 283 drives the screw to rotate to a position shown in FIG. 13, the screw completely blocks the base flow runner 282. When the handle 283 drives the screw to rotate away from the position shown in FIG. 13, the screw only blocks a part of the base flow runner 282. As a result, the cross-sectional area of the base flow runner 282 is varied, so as to vary the cross-sectional area of the inner exhausting runner 202 and/or the cross-sectional area of the inner intake runner 203.

As shown in FIG. 11, the intake device 21 is a fan module, and the decontamination device 22 includes a disinfection lamp 220, a first filter 221 and a second filter 222. The disinfection lamp 220 is disposed in the accommodating space 201 of the main casing 20. The first filter 221 is disposed on a side of the disinfection lamp 220. The second filter 222 is disposed on another side of the disinfection lamp 220. When the intake device 21 (i.e., the fan module) is activated, the intake device 21 takes the air inside the isolation space IS to the accommodating space 201 of the main casing 20 via the inner exhausting runner 202, the air flew into the isolation space IS will pass the first filter 221 located on the side of the disinfection lamp 220 in advance, such that the air flew into the accommodating space 201 is filtered by the first filter 221, disinfected by the disinfection lamp 220 and double filtered by the second filter 222 in sequence. Finally, the filtered and disinfected air is flew into the accommodating space 201 of the main casing 20 via the inner intake runner 203, so as to purify and disinfect the air inside the accommodating space 201 of the main casing 20. In the embodiment, the disinfection lamp 220 can be an Ultraviolet disinfection lamp, and the second filter 222 can be a filter complying with the requirements of High-Efficiency Particulate Air (HEPA) or Ultra Low Penetration Air (ULPA).

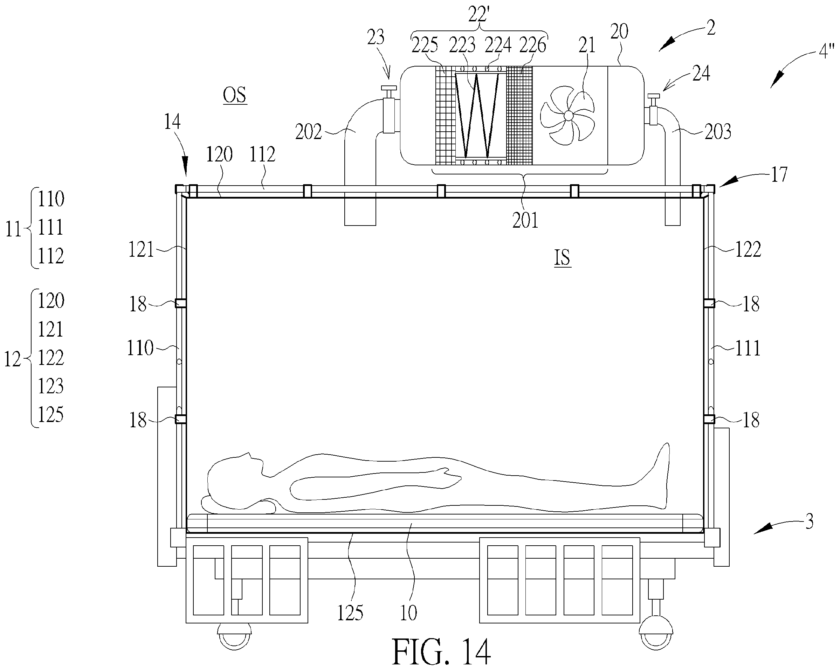

It is noticed that the decontamination device 22 of the present invention is not limited to those illustrated in figures in the embodiment. Please refer to FIG. 14. FIG. 14 is a diagram of a bed with a decontamination device 22' according to a yet embodiment of the present invention. The decontamination device 22' includes at least one photocatalyst member 223, at least one light emitting unit 224, a first filter 225 and a second filter 226. The photocatalyst member 223 is disposed the accommodating space 201 of the main casing 20. The light emitting unit 224 is disposed in the accommodating space 201 and activates the photocatalyst member 223. The first filter 225 is disposed on a side of the photocatalyst member 223, and the second filter 226 is disposed on another side of the photocatalyst member 223. When the intake device 21 (i.e., the fan module) is activated, the intake device 21 takes the air in the isolation space IS to the accommodating space 201 of the main casing 20 via the inner exhausting runner 202, the air taken into the accommodating space 201 will flow through the first filter 225 located on the side of the photocatalyst member 223, such that the air taken into the accommodating space 201 is filtered by the first filter 225. Afterwards, the air produces photocatalyst reaction with photocatalyst member 223 for disinfection and then is double filtered by the second filter 226. Finally, the filtered and disinfected air is flew into the isolation space IS, so as to purify and disinfect the air in the isolation space IS.

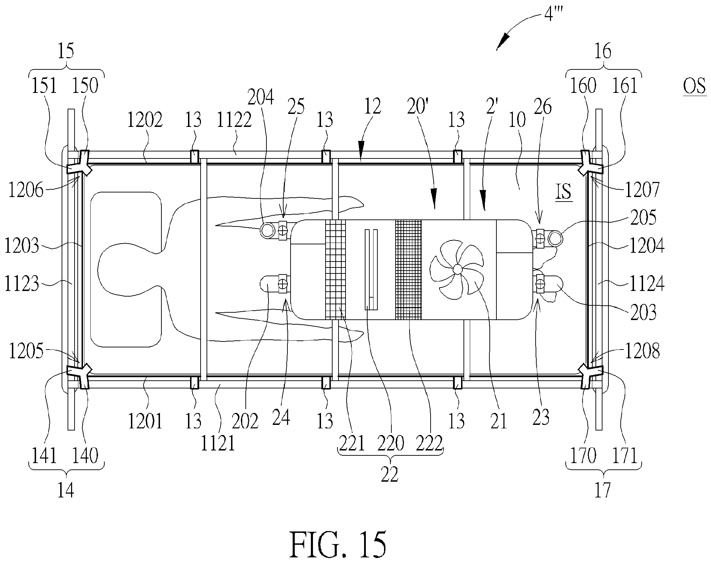

Please refer to FIG. 15. FIG. 15 is a top view of a bed set 4'" according to a fourth embodiment of the present invention. A major difference between the bed set 4'" and the aforesaid bed set 4" is that an air purifying apparatus 2' of the bed set 4'" has an inner exhausting runner 202, an inner intake runner 203, an outer exhausting runner 204 and an outer intake runner 205 formed therein. The inner exhausting runner 202 is communicated with the isolation space IS and a side of the accommodating space 201. The inner intake runner 203 is communicated with the isolation space IS and another side of the accommodating space 201. The outer exhausting runner 204 is communicated with the outer space OS and the side of the accommodating space 201. The outer intake runner 205 is communicated with the another side of the accommodating space 201 and the outer space OS. A cross-sectional area of the inner exhausting runner 202 is different from a cross-sectional area of the inner intake runner 203, and a cross-sectional area of the outer exhausting runner 204 is different from a cross-sectional area of the outer intake runner 205.

It is noticed that, in the embodiment, a sum over the cross-sectional area of the inner exhausting runner 202 and the cross-sectional area of the inner intake runner 203 is greater than a sum over the cross-sectional area of the outer exhausting runner 204 and the cross-sectional area of the outer intake runner 205. As a result, under a condition of the same intake device 21, a sum over an inner exhausting flux flew out from the isolation space IS via the inner exhausting runner 202 an inner intake flux flew into the isolation space IS via the inner intake runner 203 is greater than a sum over an outer exhausting flux flew into the accommodating space 201 via the outer exhausting runner 204 and an outer intake flux flew out from the accommodating space 201 via the outer intake runner 205. In other words, the present invention is able to adjust the sum over the inner exhausting flux and the inner intake flux to be greater than the sum over the outer exhausting flux and the outer intake flux through a design that the sum over the cross-sectional area of the inner exhausting runner 202 and the cross-sectional area of the inner intake runner 203 is greater than the sum over the cross-sectional area of the outer exhausting runner 204 and the cross-sectional area of the outer intake runner 205.

Furthermore, the air purifying apparatus 2' further includes an inner intake flow adjustment device 23, an inner exhausting flow adjustment device 24, an outer exhausting flow adjustment device 25 and an outer intake flow adjustment device 26. The inner intake flow adjustment device 23 is coupled to the inner intake runner 203 and for adjusting the inner intake flux flew into the isolation space IS via the inner intake runner 203. The inner exhausting flow adjustment device 24 is coupled to the inner exhausting runner 202 and for adjusting the inner intake flux flew out from the isolation space IS via the inner exhausting runner 202. The outer exhausting flow adjustment device 25 is coupled to the outer exhausting runner 204 and for adjusting the outer exhausting flux flew into the accommodating space 201 via the outer exhausting runner 204. The outer intake flow adjustment device 26 is coupled to the outer intake runner 205 and for adjusting the outer intake flux flew out from the accommodating space 201 via the outer intake runner 205.

In the embodiment, at least one of the inner intake flow adjustment device 23, the inner exhausting flow adjustment device 24, the outer exhausting flow adjustment device 25 and the outer intake flow adjustment device 26 is a ball valve device, a butterfly valve device, a blade valve device or a pin valve device. Structures of the ball valve device and the pin valve device are identical to those illustrated in the aforesaid embodiment(s), and related descriptions are omitted herein for simplicity.

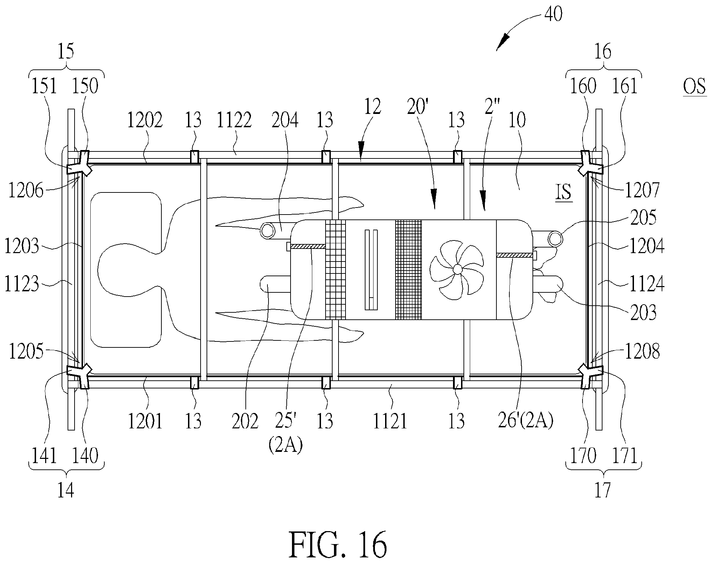

Please refer to FIG. 16. FIG. 16 is a diagram of a bed set 40 according to a fifth embodiment of the present invention. A major difference between the bed set 40 and the aforesaid bed set 4'" is that at least one of an outer exhausting flow adjustment device 25' and an outer intake flow adjustment device 26' of the bed set 40 is a flow adjustment separating member 2A. The flow adjustment separating member 2A is able to vary the cross-sectional areas of the outer exhausting runner 204 and the outer intake runner 205 in a manner of the flow adjustment separating member 2A being movable relative to the main casing 20. In the embodiment, all the outer exhausting flow adjustment device 25' and the outer intake flow adjustment device 26' are the flow adjustment separating members 2A. When the outer exhausting flow adjustment device 25' moves upwards according to figure orientation, the cross-sectional area of the outer exhausting runner 204 is getting smaller while the cross-sectional area of the inner exhausting runner 202 is getting larger. On the contrary, when the outer exhausting flow adjustment device 25' moves downwards according to figure orientation, the cross-sectional area of the outer exhausting runner 204 is getting larger while the cross-sectional area of the inner exhausting runner 202 is getting smaller. On the other hand, when outer intake flow adjustment device 26' moves upwards according to figure orientation, the cross-sectional area of the outer intake runner 205 is getting smaller while the cross-sectional area of the inner intake runner 203 is getting larger. On the contrary, when the outer intake flow adjustment device 26' moves downwards according to figure orientation, the cross-sectional area of the outer intake runner 205 is getting larger while the cross-sectional area of the inner intake runner 203 is getting smaller.

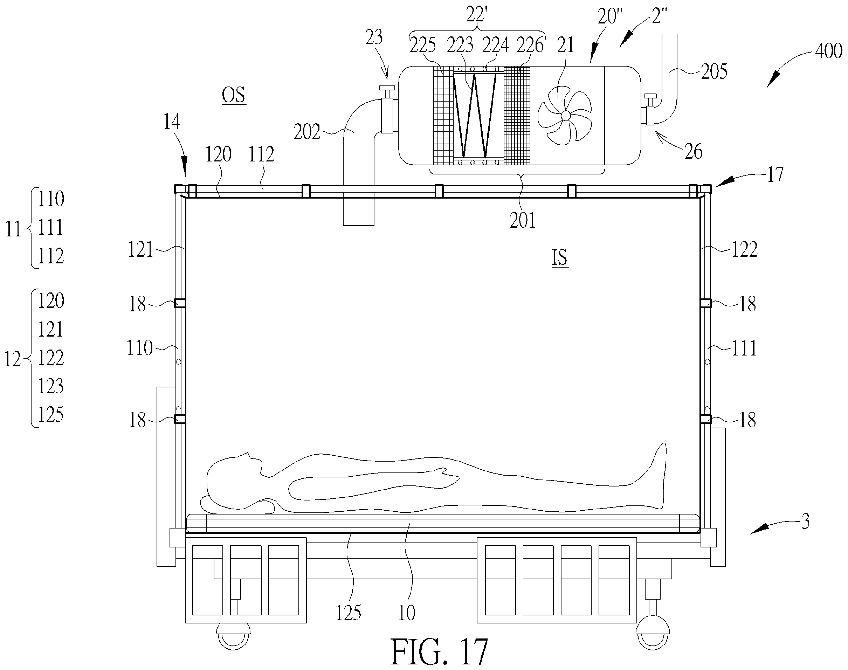

Please refer to FIG. 17. FIG. 17 is a front view of a bed set 400 according to a sixth embodiment of the present invention. A major difference between the bed set 400 and the aforesaid bed set 40 is that a main casing 20" of an air purifying apparatus 2' of a bed set 400 has an accommodating space 201, an inner exhausting runner 202 and an outer intake runner 205 formed therein. The inner exhausting runner 202 is communicated with the isolation space IS and a side of the accommodating space 201. The outer intake runner 205 is communicated with another side of the isolation space IS and the outer space OS. A cross-sectional area of the inner exhausting runner 202 is different from a cross-sectional area of the outer intake runner 205.

In such a manner, when air in the isolation space IS is flew into the accommodating space 201 via the inner exhausting runner 202, the air is purified and disinfected by the decontamination device 22 and then intake to the outer space OS via the outer intake runner 205, so as to purify and disinfect the air in the isolation space IS. In other words, the air purifying apparatus 2" of the bed set 400 has a circulation mode of "inner-exhausting-outer-intake". Components with denoted in this embodiment identical to those in the aforesaid embodiment have identical structures and functions, and further description is omitted herein for simplicity.

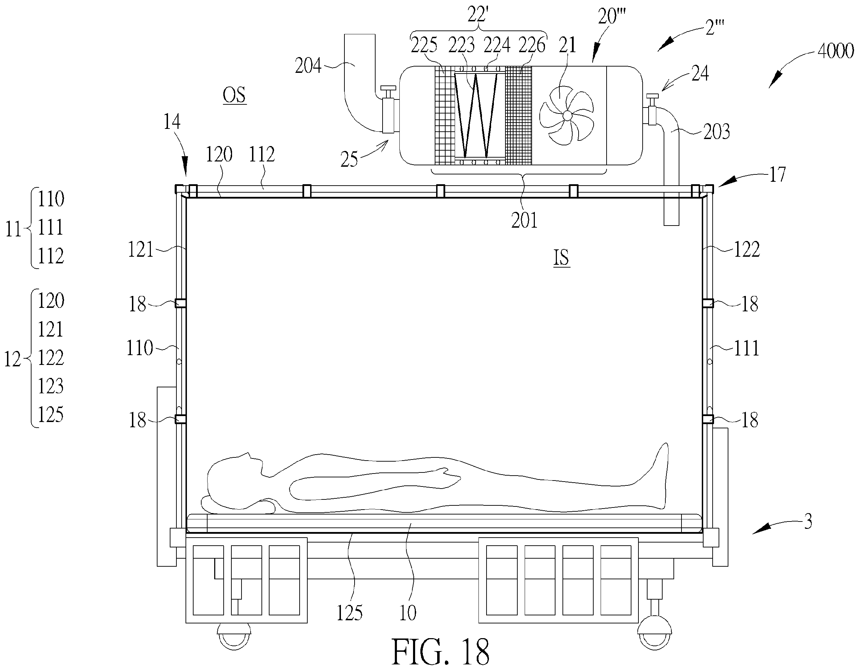

Please refer to FIG. 18. FIG. 18 is a front view of a bed set 4000 according to a seventh embodiment of the present invention. A major difference between the bed set 4000 and the aforesaid bed set 4 is that a main casing 20'" of an air purifying apparatus 2"' of the bed set 4000 has an accommodating space 201, an outer exhausting runner 204 and an inner intake runner 203 therein. The outer exhausting runner 204 is communicated with the outer space OS and a side of the accommodating space 201. The inner intake runner 203 is communicated with the isolation space IS and another side of the accommodating space 201. A cross-sectional area of the outer exhausting runner 204 is different from a cross-sectional area of the inner intake runner 203.

In such a manner, when air in the outer space OS is flew into the accommodating space 201 via the outer exhausting runner 204, the air is purified and disinfected by the decontamination device 22 and then intake to the isolation space IS via the inner intake runner 203, so as to purify and disinfect the air in the isolation space IS. In other words, the air purifying apparatus 2"' of the bed set 4000 has a circulation mode of "outer-exhausting-inner-intake". Components with denoted in this embodiment identical to those in the aforesaid embodiment have identical structures and functions, and further description is omitted herein for simplicity.

Compared to the prior art, the air purifying apparatus installed on the mask body is utilized to purify and disinfect the air in the isolation space surrounded by the mask, wherein the isolation space reduces the possibility of users (such as patients) being affected by the outer space, and is equipped with cleaning equipment for purifying and disinfecting, thereby reducing the risk of cross-infection during hospitalization.

Those skilled in the art will readily observe that numerous modifications and alterations of the device and method may be made while retaining the teachings of the invention. Accordingly, the above disclosure should be construed as limited only by the metes and bounds of the appended claims.

## Claims

1. An air purifying apparatus (2, 2', 2") for purifying air in an isolation space (IS), the isolation space (IS) being surrounded by a mask body (19), the isolation space (IS) and an outer space (OS) being bounded by the mask body (19), the air purifying apparatus (2, 2') being **characterized by**:
a main casing (20, 20') having an accommodating space (201), an inner exhausting runner (202) and an inner intake runner (203) formed therein, the inner exhausting runner (202) being communicated with the isolation space (IS) and a side of the accommodating space (201), the inner intake runner (203) being communicated with the isolation space (IS) and another side of the accommodating space (201), a cross-sectional area of the inner exhausting runner (202) being different from a cross-sectional area of the inner intake runner (203);
an intake device (21) disposed in the accommodating space (201); and
a decontamination (22, 22') device disposed in the accommodating space (201), the decontamination device (22, 22') being disposed between the intake device (21) and the inner exhausting runner (202) and for purifying and disinfecting air in the accommodating space (201).

2. The air purifying apparatus (2, 2', 2") of claim 1, further **characterized by**:
an inner intake flow adjustment device (23) coupled to the inner intake runner (203) and for adjusting an inner intake flux flew into the isolation space (IS) via the inner intake runner (203); or
an inner exhausting flow adjustment device (24) coupled to the inner exhausting runner (202) and for adjusting the inner exhausting flux flew out from the isolation space (IS) via the inner exhausting runner (202).

3. The air purifying apparatus (2, 2', 2") of claim 2, **characterized in that** at least one of the inner intake flow adjustment device (23) and the inner exhausting flow adjustment device (24) comprises:
a base (270) having a base flow runner (272) therein; and
a rotating valve member (271) disposed in the base flow runner (272), the rotating valve member (271) varying a cross-sectional area of the base flow runner (272) in a rotating manner.

4. The air purifying apparatus (2, 2', 2") of claim 2, **characterized in that** at least one of the inner intake flow adjustment device (23) and the inner exhausting flow adjustment device (24) comprises:
a base (280) having a base flow runner (282) therein; and
a linear valve member (281) disposed in the base flow runner (282), the linear valve member (281) varying a cross-sectional area of the base flow runner (282) in a linearly moving manner.

5. The air purifying apparatus (2', 2") of claim 1, **characterized in that** the main casing (20') further has an outer exhausting runner (204) and an outer intake runner (205), the outer exhausting runner (204) is communicated with the outer space (OS) and the side of the accommodating space (201), the outer intake runner (205) is communicated with the another side of the accommodating space (201) and the outer space (OS), a cross-sectional area of the outer exhausting runner (204) is different from a cross-sectional area of the outer intake runner (205).

6. The air purifying apparatus (2', 2") of claim 5, further **characterized by**:
an outer exhausting flow adjustment device (25, 25') coupled to the outer exhausting runner (204) and for adjusting an outer exhausting flux flew into the accommodating space (201) via the outer exhausting runner (204); or
an outer intake flow adjustment device (26, 26') coupled to the outer intake runner (205) and for adjusting the outer intake flux flew out from the accommodating space (201) via the outer intake runner (205).

7. The air purifying apparatus (2') of claim 6, **characterized in that** at least one of the outer exhausting flow adjustment device (25) and the outer intake flow adjustment device (26) comprises:
a base (270) having a base flow runner (272) therein; and
a rotating valve member (271) disposed in the base flow runner (272), the rotating valve member (271) varying a cross-sectional area of the base flow runner (272) in a rotating manner.

8. The air purifying apparatus (2') of claim 6, **characterized in that** at least one of the outer exhausting flow adjustment device (25) and the outer intake flow adjustment device (26) comprises:
a base (280) having a base flow runner (282) therein; and
a linear valve member (281) disposed in the base flow runner (282), the linear valve member (281) varying a cross-sectional area of the base flow runner (282) in a linearly moving manner.

9. The air purifying apparatus (2") of claim 6, **characterized in that** at least one of the outer exhausting flow adjustment device (25') and the outer intake flow adjustment device (26') is a flow adjustment separating member (2A), the flow adjustment separating member (2A) being configured to vary the cross-sectional area of the outer exhausting runner (204) and the cross-sectional area of the outer intake runner (205) in a manner of the flow adjustment separating member (2A) being movable relative to the main casing (20').

10. The air purifying apparatus (2, 2', 2") of any of claims 1-9, **characterized in that** the intake device (21) is a fan module.

11. The air purifying apparatus (2, 2', 2") of any of claims 1-10, **characterized in that** the decontamination device (22) comprises:
a disinfection lamp (220) disposed in the accommodating space (201), the disinfection lamp (220) being configured to disinfect the air in the accommodating space (201);
a first filter (221) disposed on a side of the disinfection lamp (220), the first filter (221) being configured to filter the air in the accommodating space (201); and
a second filter (222) disposed on another side of the disinfection lamp (220), the second filter (222) being configured to double filter the air filtered by the first filter (221).

12. The air purifying apparatus (2, 2', 2") of claim 11, **characterized in that** the second filter (222) is a filter complying with the requirements of High-Efficiency Particulate Air (HEPA) or Ultra Low Penetration Air (ULPA).

13. The air purifying apparatus (2") of any of claims 1-10, **characterized in that** the decontamination device (22') comprises:
at least one photocatalyst member (223) disposed the accommodating space (201);
at least one light emitting unit (224) disposed in the accommodating space (201) and configured to activate the at least one photocatalyst member (223) to produce photocatalyst reaction;
a first filter (225) disposed on a side of the photocatalyst member (223) and configured to filter the air in the accommodating space (201); and
a second filter (226) disposed on another side of the photocatalyst member (223), the second filter (226) being configured to double filter the air filtered by the first filter (225).

14. An air purifying apparatus (2"') for purifying air in an isolation space (IS), the isolation space (IS) being surrounded by a mask body (19), the isolation space (IS) and an outer space (OS) being bounded by the mask body (19), the air purifying apparatus (2"') being **characterized by**:
a main casing (20"') having an accommodating space (201), an outer exhausting runner (204) and an inner intake runner (203) formed therein, the outer exhausting runner (204) being communicated with the outer space (OS) and a side of the accommodating space (201), the inner intake runner (203) being communicated with the isolation space (IS) and another side of the accommodating space (201), a cross-sectional area of the outer exhausting runner (204) being different from a cross-sectional area of the inner intake runner (203);
an intake device (21) disposed in the accommodating space (201); and
a decontamination device (22') disposed in the accommodating space (201), the decontamination device (22') being disposed between the intake device (21) and the outer exhausting runner (204) and for purifying and disinfecting air in the accommodating space (201).

15. The air purifying apparatus (2'") of claim 14, further **characterized by**:
an inner intake flow adjustment device (24) coupled to the inner intake runner (203) and for adjusting an inner intake flux flew into the isolation space (IS) via the inner intake runner (203); or
an outer exhausting flow adjustment device (25) coupled to the outer exhausting runner (204) and for adjusting an outer exhausting flux flew into the accommodating space (201) via the outer exhausting runner (204).

16. An air purifying apparatus (2") for purifying air in an isolation space (IS), the isolation space (IS) being surrounded by a mask body (19), the isolation space (IS) and an outer space (OS) being bounded by the mask body (19), the air purifying apparatus (2") being **characterized by**:
a main casing (20") having an accommodating space (201), an inner exhausting runner (202) and an outer intake runner (205) formed therein, the inner exhausting runner (202) being communicated with the isolation space (IS) and a side of the accommodating space (201), the outer intake runner (205) being communicated with the outer space (OS) and another side of the accommodating space (201), a cross-sectional area of the inner exhausting runner (202) is different from a cross-sectional area of the outer intake runner (205);
an intake device (21) disposed in the accommodating space (201); and
a decontamination device (22') disposed in the accommodating space (201), the decontamination device (22') being disposed between the intake device (21) and the inner exhausting runner (202) and for purifying and disinfecting air in the accommodating space (201).

17. The air purifying apparatus (2") of claim 16, further **characterized by**:
an inner exhausting flow adjustment device (23) coupled to the inner exhausting runner (202) and for adjusting the inner exhausting flux flew out from the isolation space (IS) via the inner exhausting runner (202); or
an outer intake flow adjustment device (26) coupled to the outer intake runner (205) and for adjusting the outer intake flux flew out from the accommodating space (201) via the outer intake runner (205).
